Europäisches Patentamt

European Patent Office

Office européen des brevets

(11) Numéro de publication: **0 430 839 A1**

## (12) DEMANDE DE BREVET EUROPEEN

(21) Numéro de dépôt: 90470056.4

(51) Int. Cl.5: **A61C 1/18**

(22) Date de dépôt: **08.10.90**

(30) Priorité: **27.11.89 FR 8915760**

(43) Date de publication de la demande:
**05.06.91 Bulletin 91/23**

(84) Etats contractants désignés:
**AT CH DE IT LI Bulletin 1**

(71) Demandeur: **MICRO MEGA S.A.**
**5-12, rue du Tunnel**
**F-25000 Besançon(FR)**

(72) Inventeur: **Jacoulet, Jean-Paul**
**7 bis avenue de la Vaite**
**F-25000 Besancon(FR)**
Inventeur: **Pernot, Jacques**
**Chemin de Merey, Vieilley**
**F-25870 Geneuille(FR)**

(74) Mandataire: **Poupon, Michel**
**3, rue Thiers B.P. 247**
**F-88007 Epinal Cédex(FR)**

(54) Dispositif de raccordement d'une pièce à main ou contre-angle de dentisterie sur un support.

(57) Dispositif de raccordement d'une pièce à main ou contre-angle de dentisterie sur le support les raccordant à l'unité dentaire, tel qu'un moteur, comprenant des moyens de fourniture d'eau et d'air pour l'arrosage par spray du point de travail de l'instrument qu'il supporte et éventuellement des moyens d'alimentation en courant électrique d'ampoule situées dans la tête de la pièce à main ou du contre-angle aux fins d'éclairage du point de travail ainsi que éventuellement des moyens d'alimentation pour fibres optiques, caractérisé en ce que la face arrière de la pièce à main (1) est pourvue d'une bague (6) en matériau isolant faisant saillie et que le moteur (2) possède une forme creuse annulaire (7) recevant ladite bague lorsque la pièce à main est montée sur le moteur.

FIG. 1

EP 0 430 839 A1

# DISPOSITIF DE RACCORDEMENT D'UNE PIÈCE À MAIN OU CONTRE-ANGLE DE DENTISTERIE SUR UN SUPPORT

La présente invention a pour objet un dispositif de raccordement d'une pièce à main ou contre-angle de dentisterie sur le support les raccordant à l'unité dentaire tel qu'un moteur, comprenant des moyens de fourniture d'eau et d'air pour l'arrosage par spray du point de travail de l'instrument qu'il supporte et éventuellement des moyens d'alimentation en courant électrique d'ampoules situées dans la pièce à main ou le contre-angle aux fins d'éclairage du point de travail ainsi que éventuellement des moyens d'alimentation pour fibres optiques.

L'invention a également pour objet à titre individuel les pièces à main et les supports ainsi équipés.

La technique des pièces à main de dentisterie pourvues de dispositifs de spray air-eau intégré et/ou de dispositif d'éclairage du lieu de travail de l'instrument est maintenant largement diffusée dans le monde dentaire.

L'introduction de ces fonctions complémentaires a nécessité la création de dispositif de raccordement entre par exemple les moteurs et les pièces à main ou contre-angle.

A une époque où les cordons d'alimentation reliant la pièce à main à l'unit dentaire étaient d'une rigidité trop importante, on a cherché à réaliser des dispositifs rotatifs qui induisent l'utilisation de raccordements étanches à base de joints toriques d'où un risque d'usure ou de contacts électriques frottants qui sont aussi une source de panne par usure.

Le brevet français 2 525 467 présente un dispositif dans lequel il n'y a pas rotation du contre-angle par rapport à la pièce à main, mais avec l'inconvénient que les conduits de spray passent du moteur eitre des joints toriques qui à chaque mise en place du contre-angle sur le moteur subissent une agression induisant à terme un risque de fuite.

L'invention a pour but de remédier à ces inconvénients des dispositifs de raccordement de l'art antérieur, en prévoyant en outre que les moteurs spéciaux modifiés conformément à l'invention puissent recevoir de manière compatible en fonctionnement de celles-ci des pièces à main usuelles non conformes à l'invention.

Conformément à l' ivention, ce résultat est obtenu avec un dispositif de raccordement d'une pièce à rain ou contre-angle de dentisterie sur le support les raccordant à l'unité dentaire, tel qu'un moteur, comprenant des moyens de fourniture d'eau et d'air pour l'arrosage par spray du point de travail de l'instrument qu'il supporte et éventuellement des moyens d'alimentation en courant électrique d'ampoules situées dans la pièce à main ou le contre-angle aux fins d'éclairage du point de travail ainsi que éventuellement des moyens d'alimentation pour fibres optiques, caractérisé en ce que la face arrière de la pièce à main est pourvue d'une bague en matériau isolant faisant saillie et que le moteur possède une forme creuse annulaire recevant ladite bague lorsque la pièce à main est montée sur le moteur.

Selon un mode avantageux de mise en oeuvre, la bague est pourvue d'une encoche et le moteur d'un ergot destinés à s'interpénétrer pour assurer le positionnement relatif angulaire des deux éléments et les canaux d'eau et d'air débouchent de la bague de façon longitudinale, le moteur ayant en vis-à-vis des tubes s'engageant dans les trous débouchant de la bague.

On comprendra mieux l'invention à l'aide de la description faite ci-après d'un mode de mise en oeuvre donné à titre d'exemple non limitatif en référence aux schémas annexés dans lesquels :

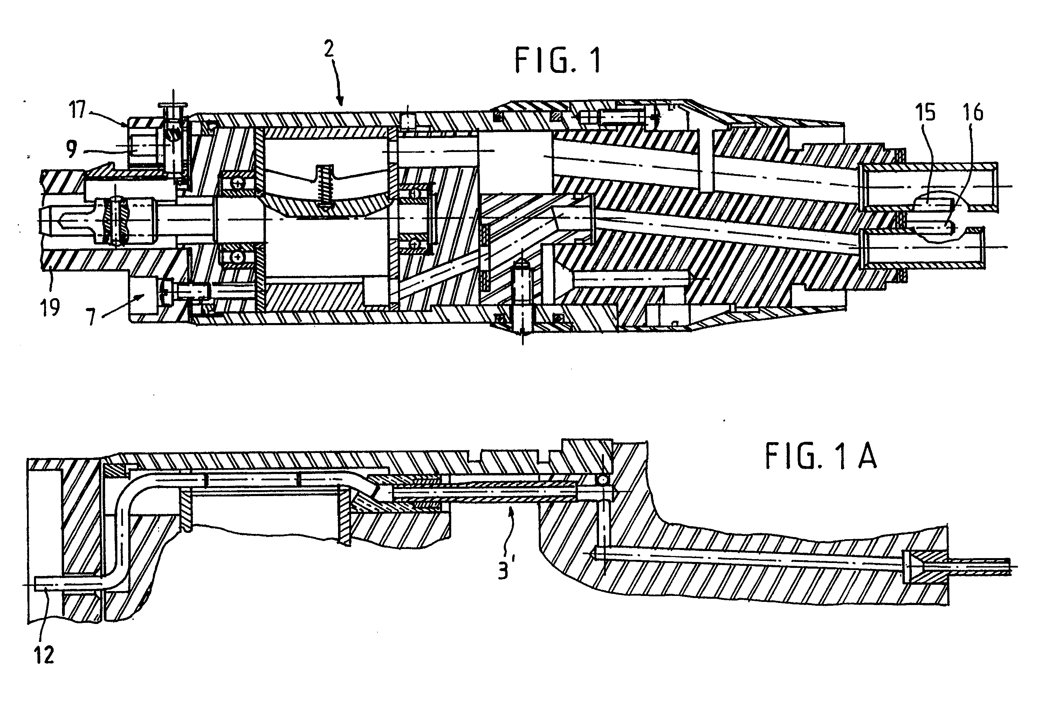

- la figure 1 est une vue en coupe longitudinale d'un moteur avec dispositif de raccordement conforme à l'invention avec détails des cheminements de spray en 1A et électrique en 1B ;
- les figures 2, 2A et 2B sont les vues correspondantes d'une pièce à main avec dispositif de raccordement conforme à l'invention avec une figure 2C qui est une vue de la pièce à main côté raccordement.

Le dispositif de l'invention est destiné à raccorder une pièce à main ou contre-angle généralement référencé (1) à un support (2) en l'occurrence un moteur. Le support pourrait être de toute autre nature, relié à une unité dentaire, sans sortir du cadre de l'invention.

On ne décrira pas plus en détail les moyens connus de l'homme de l'art internes à la pièce (1) et au moteur (2) qui permettent le passage à travers ceux-ci de conduites d'air et d'eau (3,3') et d'électricité (4,4').

Conformément à l'invention, on prévoit que la face arrière (5) de la pièce à main comporte une bague (6) en matériau isolant, de préférence un matériau moulable, faisant saillie par rapport au plan de ladite face arrière et que le support, à savoir ici le moteur (2), possède une forme réceptrice creuse annulaire (7) correspondante recevant ladite bague lorsque la pièce à main est enfichée sur le moteur.

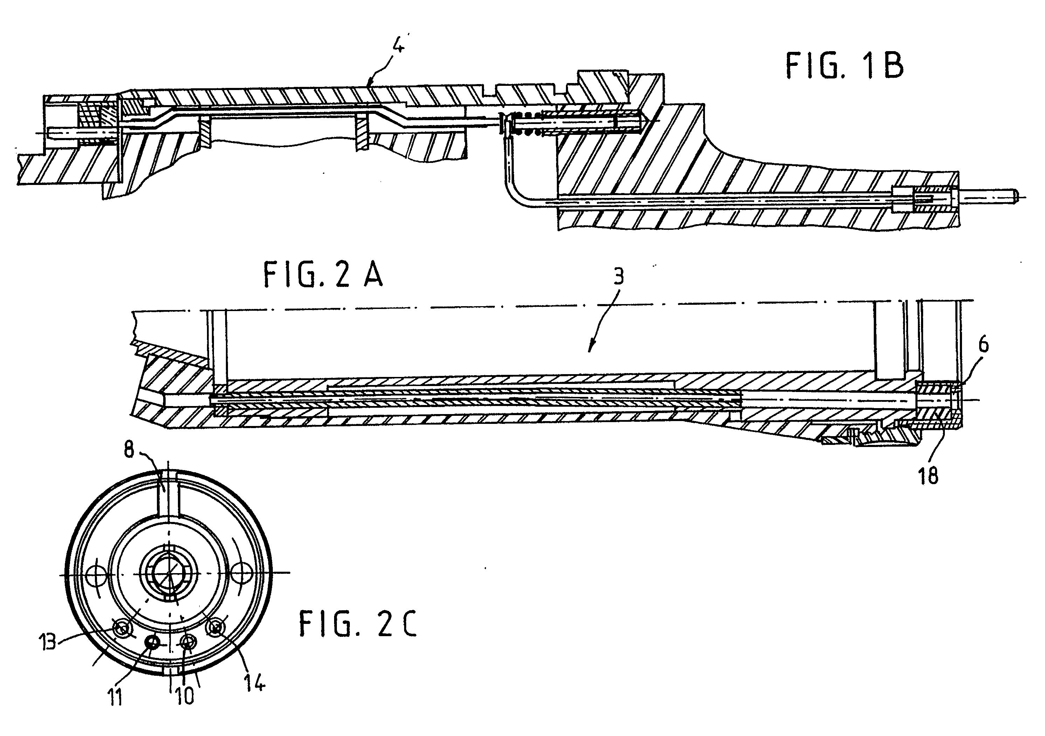

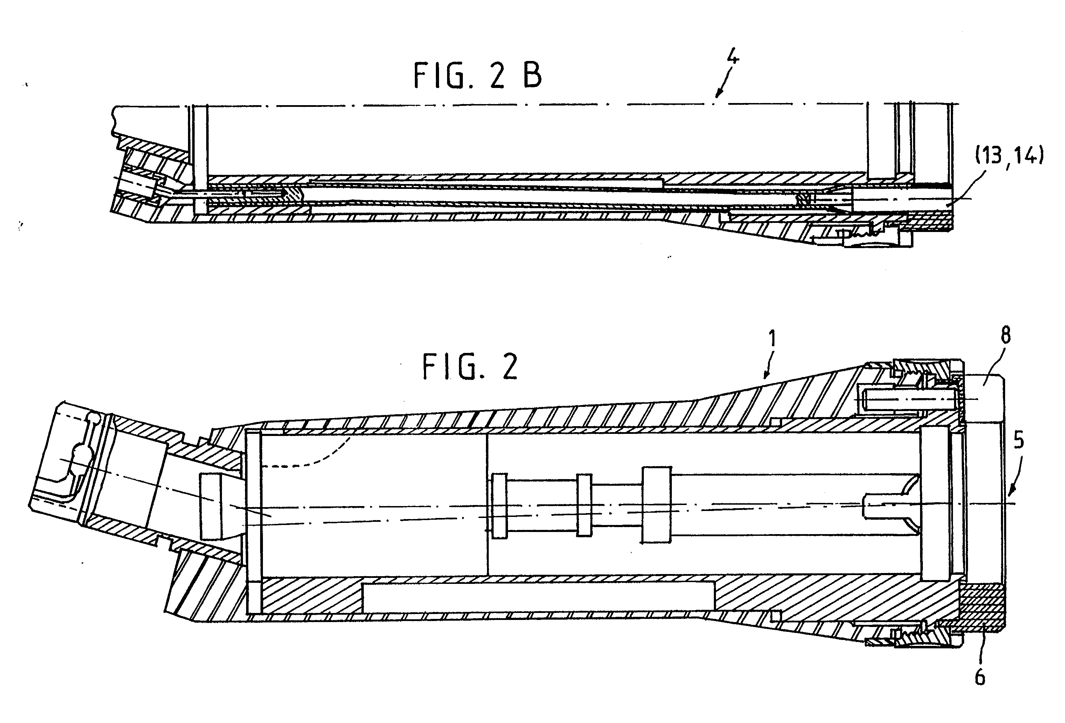

Avantageusement, la bague (6) est pourvue d'une encoche (8), visible figure 2C, et le moteur d'un ergot (9) destinés à s'interpénétrer pour assurer le juste positionnement relatif angulaire des deux pièces (1) et (2).

Ainsi les canaux respectifs d'eau (10) et d'air (11) débouchent de la bague longitudinalement, le moteur comportant, en vis-à-vis, des tubes (12) s'engageant dans les sorties (10) et (11).

Selon une caractéristique important de l'invention, la pièce à main est pourvue de deux contacts femelles (13,14) positionnés axialement. Le moteur possède deux contacts mâles (15,16) sous forme de piges venant pénétrer lesdits contacts femelles et assurant donc la liaison électrique.

Pour des raisons de protection des tubes et des contacts du moteur, on prévoiera que ceux-ci ne débordent pas de la gorge annulaire (7). Ceci permet également d'y adapter toute autre pièce à main classique.

Plus précisément, on prévoiera que la face (17) du moteur devant recevoir la pièce à main est telle que le moteur puisse recevoir, sans destruction des tubes et/ou des contacts ou établissement d'un court-circuit entre contacts, des pièces à main sans spray ni lumière dont l'accouplement est suivant la norme ISO 3964.

Selon un caractéristique avantageuse de mise en oeuvre de l'invention, on prévoiera que l'étanchéité entre les tubes (12) du moteur et les orifices air/eau (10,11) de la pièce à main est assurée par un joint cylindrique (18) logé dans la bague (6) et comprimé par elle lors du montage.

Enfin, on prévoiera que le moteur soit équipé d'une douille de raccordement (19) suivant la norme ISO 3964.

La douille fait corps avec la face frontale du moteur et cette pièce unique est réalisée en matériau moulable isolant.

## Revendications

1. Dispositif de raccordement d'une pièce à main ou contre-angle de dentisterie sur le support les raccordant à l'unité dentaire, tel qu'un moteur, comprenant des moyens de fourniture d'eau et d'air pour l'arrosage par spray du point de travail de l'instrument qu'il supporte et éventuellement des moyens d'alimentation en courant électrique d'ampoule situées dans la tête de la pièce à main ou du contre-angle aux fins d'éclairage du point de travail ainsi que éventuellement des moyens d'alimentation pour fibres optiques, caractérisé en ce que la face arrière de la pièce à main (1) est pourvue d'une bague (6) en matériau isolant faisant saillie et que le moteur (2) possède une forme creuse annulaire (7) recevant ladite bague lorsque la pièce à main est montée sur le moteur.

2. Dispositif de raccordement selon la revendication 1, caractérisé en ce que la bague (6) est pourvue d'une encoche (8) et le moteur d'un ergot (9) destinés à s'interpénétrer pour assurer le positionnement relatif angulaire des deux éléments et que les canaux d'eau (10) et d'air (11) débouchent de la bague de façon longitudinale, le moteur ayant en vis-à-vis des tubes (12) s'engageant dans les trous (10,11) débouchant de la bague.

3. Dispositif de raccordement selon l'une des revendications 1 et 2, caractérisé en ce que la pièce à main est pourvue de deux contacts femelles (13,14) positionnés axialement et que le moteur possède deux contacts mâles (15,16) sous forme de piges venant pénétrer lesdits contacts femelles et assurant la liaison électrique.

4. Dispositif de raccordement selon l'une quelconque des revendications 1 à 3, caractérisé en ce que les tubes et les contacts du moteur ne débordent pas de la gorge annulaire.

5. Dispositif de raccordement selon l'une des revendications 1 à 4, caractérisé en ce que la face (17) du moteur devant recevoir la pièce à main est telle que le moteur puisse recevoir sans destruction des tubes et/ou des contacts ou établissement d'un court-circuit entre contacts, des pièces à main sans spray ni lumière dont l'accouplement est suivant la norme ISO 3964.

6. Dispositif de raccordement selon l'une des revendications 1 à 5, caractérisé en ce que l'étanchéité entre les tubes (12) du moteur et les orifices air/eau (10,11) de la pièce à main est assurée par un joint cylindrique (18) logé dans la bague (6) et comprimé par elle lors du montage.

7. Dispositif de raccordement selon l'une des revendications 1 à 6, caractérisé en ce que le moteur est équipé d'une douille de raccordement (19) suivant la norme ISO 3964.

8. Dispositif de raccordement selon la revendication 7, caractérisé en ce que la douille (19) fait corps avec la face frontale du moteur et que cette pièce unique est réalisée en matériau moulable isolant.

9. Pièce à main ou support de pièce à main, caractérisé en ce qu'il comporte un dispositif de raccordement selon l'une quelconque des revendications 1 à 8.

## FIG. 1

## FIG. 1 A

FIG. 1B

FIG. 2 A

FIG. 2 C

EP 0 430 839 A1

FIG. 2 B

4

(13,14)

FIG. 2

1

8

5

6

EP 0 430 839 A1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | FR-A-2 584 918   (MICRO MEGA)<br>* En entier *<br>– – – | 1-9 | A 61<br>C 1/18 |
| D,A | GB-A-2 118 838   (KALTENBACH & VOIGT)<br>* Abrégé *<br>– – – | 1 | |
| A | DE-A-3 339 650   (SIEMENS)<br>* Page 13, ligne 1 - page 14, ligne 7; figures 4-6,15,16 *<br>– – – | 1,2,4,9 | |
| A | US-A-4 324 548   (NILES)<br>* Abrégé; figures 1,3 *<br>– – – – – | 1,2 | |

| DOMAINES TECHNIQUES RECHERCHES (Int. Cl.5) |
|---|
| A 61 C |

Le présent rapport de recherche a été établi pour toutes les revendications

| Lieu de la recherche | Date d'achèvement de la recherche | Examinateur |
|---|---|---|
| La Haye | 01 mars 91 | KOUSOURETAS I. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention

E : document de brevet antérieur, mais publié à la date de dépôt ou après cette date
D : cité dans la demande
L : cité pour d'autres raisons

& : membre de la même famille, document correspondant